# EUROPÄISCHE PATENTSCHRIFT

(11) **EP 0 691 855 B1**
(45) Veröffentlichungstag und Bekanntmachung des Hinweises auf die Patenterteilung: **19.05.1999**
(21) Anmeldenummer: 94913431.6
(22) Anmeldetag: 06.02.1993
(51) Int. Cl.: A61L 2/26, G09F 3/03

(54) **STERILISIERBEHÄLTER FÜR CHIRURGISCHE INSTRUMENTE ODER DERGLEICHEN**
STERILISATION CONTAINER FOR SURGICAL INSTRUMENTS OF THE LIKE
RECIPIENT A STERILISER POUR INSTRUMENTS CHIRURGICAUX OU ANALOGUES

(30) Priorität: 16.10.1992 DE 9213983 U; 02.12.1992 DE 9216378 U
(43) Veröffentlichungstag der Anmeldung: 17.01.1996
(73) Patentinhaber: Aesculap AG & Co. KG, 78532 Tuttlingen (DE)
(72) Erfinder: GABELE, Lorenz, D-88605 Sauldorf (DE); KULOW, Uwe, D-78532 Tuttlingen (DE); SCHWANKE, Wolfgang, D-72379 Weilheim (DE); TASCHNER, Wolfgang, D-78532 Tuttlingen (DE)
(74) Vertreter: Böhme, Ulrich, Dr. Dipl.-Phys.
(86) Internationale Anmeldenummer: EP9300283
(87) Internationale Veröffentlichungsnummer: WO9408632

(56) Entgegenhaltungen:
- WO-A-92/06899
- DE-A- 3 316 141
- DE-A- 3 541 309

## Beschreibung

Die Erfindung betrifft einen Sterilisierbehälter für chirurgische Instrumente oder dergleichen, mit einem Unterteil, einem abgedichtet auf diesem aufsetzbaren Deckel, einem den Deckel am Unterteil festlegenden Riegel, der zwischen einer Schließstellung und einer Offenstellung verschwenkbar ist, und mit einem kartenähnlichen Sicherungselement, das am Behälter festlegbar ist und nahe der Schließstellung des Riegels in die Bewegungsbahn des Riegels eintaucht.

Ein solcher Sterilisierbehälter ist aus dem deutschen Patent 35 41 309 bekannt. Das kartenähnliche Sicherungselement wird normalerweise gleichzeitig als Datenträger benutzt, auf dem wesentliche Daten des Behälterinhaltes und der Behandlungsart aufgezeichnet werden. Die bekannte Ausführung hat den Vorteil, daß ein unautorisiertes Öffnen des Riegels nach einem Sterilisiervorgang das Sicherungselement beschädigt, so daß man an dieser Beschädigung jedes unautorisierte Öffnen des Behälters nachträglich erkennen kann. Die Beschädigung besteht bei bekannten Sicherungselementen in einer Einstanzung, die zwar an dem herausgenommenen Sicherungselement gut erkennbar ist, die aber bei einem eingeschobenen und am Behälter gehaltenen Sicherungselement schlecht erkennbar ist, da sie von dem am Riegel gehaltenen Stanzelement überdeckt wird.

Es ist Aufgabe der Erfindung, einen Sterilisierbehälter der eingangs beschriebenen Art so weiterzubilden, daß man an dem eingeschobenen Sicherungselement sofort erkennt, ob eine unautorisierte Öffnung des Behälters stattgefunden hat oder nicht.

Diese Aufgabe wird bei einem Sterilisierbehälter der eingangs beschriebenen Art erfindungsgemäß dadurch gelöst, daß der in die Bewegungsbahn des Riegels eintauchende Teil des Sicherungselementes aus der Ebene des Sicherungselementes elastisch abbiegbar ist und nur so tief in die Bewegungsbahn des Riegels eintaucht, daß er beim Verschwenken des Riegels in Offenstellung hinter den Riegel zurückgleitet und beim Schließen des Riegels von diesem mitgenommen und in der Schließstellung des Riegels von diesem abgedeckt wird. Es wird also ein Teil des Sicherungselementes, welcher nach dem Verschließen des Behälters vor dem Riegel liegt, beim Öffnen des Riegels von diesem nach vorne abgebogen und gleitet dann hinter den Riegel, so daß er bei einem erneuten Schließen des Riegels von diesem verdeckt wird. Dadurch ist auf den ersten Blick erkennbar, ob eine Öffnung des Riegels stattgefunden hat. Dies ist nur dann ausgeschlossen, wenn der in die Bewegungsbahn des Riegels eintauchende Teil vor dem Riegel liegt und nicht von ihm abgedeckt wird.

Günstig ist es, wenn der in die Bewegungsbahn des Riegels eintauchende Teil ein seitlich von dem kartenähnlichen Sicherungselement abstehender Lappen ist.

Bei einer bevorzugten Ausführungsform kann vorgesehen sein, daß neben dem Riegel eine Halterung für das kartenähnliche Sicherungselement angeordnet ist, in welche das Sicherungselement nach dem Schließen des Riegels derart einlegbar oder einschiebbar ist, daß der in die Bewegungsbahn des Riegels eintauchende Teil unmittelbar vor dem Riegel positioniert ist. Die Halterung wird also beispielsweise unmittelbar neben dem Riegel an der Seitenwand des Unterteils festgelegt, so daß sie sich im wesentlichen in derselben Ebene befindet wie der geschlossene Riegel.

Besonders vorteilhaft ist es, wenn der in die Bewegungsbahn des Riegels eintauchende Teil nach dem Einlegen oder Einschieben in die Halterung durch die Anlage am Riegel nach vorne aus der Ebene des kartenförmigen Sicherungselementes abgebogen ist. Dadurch ist dieser abgebogene Teil des Sicherungselementes besonders auffällig und sichtbar, so daß man auf den ersten Blick erkennen kann, daß der Riegel bisher noch nicht geöffnet war.

Es kann dabei vorgesehen sein, daß der Riegel eine Umlenkfläche aufweist, an der der in die Bewegungsbahn des Riegels eintauchende Teil des kartenförmigen Sicherungselementes beim Einlegen oder Einschieben in die Halterung vor den Riegel gelenkt wird, so daß der Benutzer des Behälters beim Einlegen nicht besonders darauf achten muß, daß ein Teil des Sicherungselementes sich vor den Riegel legt, sondern dies erfolgt beim Einlegen automatisch durch diese Umlenkfläche.

Die Halterung kann auf ihrer dem Riegel abgewandten Seite einen Einführschlitz für das Sicherungselement aufweisen, wobei vorzugsweise der Einführschlitz gegenüber der Ebene, in welcher das Sicherungselement im Halter positionierbar ist, seitlich versetzt ist. Diese seitliche Versetzung führt dazu, daß das kartenförmige Sicherungselement nur unter Verformung und Verbiegung in den Halter eingeschoben werden kann und nach dem Einschieben in den Halter nicht mehr in umgekehrter Richtung aus diesem herausgeschoben werden kann, da das elastische Sicherungselement beim Verschieben in umgekehrter Richtung nicht in den seitlich versetzten Schlitz gelangen kann. Dies ist eine Sicherung dagegen, daß das Sicherungselement aus der Bewegungsbahn des Riegels herausgeschoben wird, um den Riegel unautorisiert und unkontrolliert zu öffnen. Nach dem Einschieben des Sicherungselementes bleibt dieses bis zum Abschluß des Vorganges in der Halterung und kann nur nach Öffnung des Riegels aus dem Halter herausgezogen werden.

Bei einer besonders bevorzugten Ausführungsform ist vorgesehen, daß der in die Bewegungsbahn des Riegels eintauchende Teil des kartenförmigen Sicherungselementes mittels einer Sollbruchstelle und mittels einer festeren flexiblen Verbindung mit dem übrigen Sicherungselement verbunden ist und daß die Sollbruchstelle so ausgebildet und positioniert ist, daß sie bricht, wenn der Riegel den vor ihm liegenden Teil des Sicherungselementes beim Verschwenken in Offenstellung mitnimmt, während die festere Verbindung erhalten bleibt. Diese spezielle Ausgestaltung führt einerseits dazu, daß durch Beschädigung der Sollbruchstelle eine dauerhafte Veränderung an dem Sicherungselement erfolgt, sobald der Riegel einmal in Offenstellung verschwenkt wird, während andererseits der in die Bewegungsbahn des Riegels eintauchende Teil des Sicherungselementes mit diesem verbunden bleibt, so daß beim erneuten Verschließen des Riegels dieser Teil trotz der Beschädigung der Sollbruchstelle hinter den Riegel mitgenommen und von diesem verdeckt wird.

Der in die Bewegungsbahn des Riegels eintauchende Teil kann dazu über zwei verschieden breite Verbindungsstege mit dem übrigen Sicherungselement verbunden sein, es ist bei einer anderen Ausführungsform auch möglich, daß im Sicherungselement neben dem in die Bewegungsbahn des Riegels eintauchenden Teil eine Durchbrechung angeordnet ist, die zwischen dem in die Bewegungsbahn eintauchenden Teil und dem übrigen Sicherungselement einen breiten und einen schmalen Verbindungssteg ausbildet.

Dabei ist es besonders vorteilhaft, wenn der Riegel einen seitlichen Fortsatz trägt, der beim Verschwenken des Riegels in Offenstellung zwischen die Verbindungsstege eingreift und beim weiteren Verschwenken dadurch den schmaleren Verbindungssteg aufreißt.

Bei einer anderen Ausführungsform kann vorgesehen sein, daß der Riegel ein Markierungsglied trägt, das beim Verschwenken des Riegels aus der Schließstellung in die Offenstellung das Sicherungselement durchdringt und dadurch beschädigt. Eine solche Beschädigung des Sicherungselementes, die aus der DE-PS 35 41 309 an sich bekannt ist, kann ebenfalls dazu dienen, das Sicherungselement zu markieren, sobald der Riegel einmal in die Offenstellung verschwenkt wird. Trotzdem bleibt auch bei dieser Lösung das in die Bewegungsbahn eintauchende Teil mit dem Sicherungselement verbunden, so daß man auch bei eingeschobenem Sicherungselement sofort am Behälter erkennen kann, ob der Riegel geöffnet worden ist oder nicht.

Bei einer weiteren bevorzugten Ausführungsform kann vorgesehen sein, daß neben dem vor dem Riegel positionierten Teil des Sicherungselementes ein Niederhalter angeordnet ist, der sich vor dem Sicherungselement befindet und dieses in Richtung auf den Sterilisierbehälter festlegt. Bei einer solchen Ausgestaltung wird das Sicherungselement in einem Teilbereich nahe der Wand des Sterilisierbehälters gehalten, während ein anderer Teil vor dem Riegel liegt. Wenn das Sicherungselement in diesem Bereich beschädigt ist, beispielsweise durch eine unsachgemäße vorherige Öffnung, führt dies dazu, daß das Sicherungselement im Bereich vor dem Riegel schräg nach vorne absteht.

Besonders vorteilhaft ist es, wenn der Niederhalter das Sicherungselement in einer Position festlegt, in der das Sicherungselement zwischen dem vom Niederhalter überdeckten Bereich und dem vor dem Riegel liegenden Bereich vom Sterilisierbehälter nach außen weisend vorgespannt ist. Ein unbeschädigtes Sicherungselement wird durch die Vorspannung nicht wesentlich verformt, da das Sicherungselement durch den Halter im wesentlichen parallel zur Einlegeebene des Sicherungselementes festgelegt wird. Wenn jedoch ein Teilbereich des Sicherungselementes ausgerissen oder ausgestanzt ist, so daß dieser Teil nur noch einseitig am Sicherungselement gehalten ist, dann führt diese Vorspannung zwangsläufig zu einem nach außen gerichteten Abstehen dieses Bereiches aus der Ebene des Sicherungselementes, und dies ist für die Bedienungsperson sofort erkennbar.

Der Niederhalter kann ein Teil des Riegels sein.

Besonders vorteilhaft ist es, wenn im Riegel ein parallel zur Einschubrichtung des Sicherungselementes verlaufender Spalt angeordnet ist, an dessen einer Seite der das Sicherungselement überdeckende Niederhalter und an dessen anderer Seite ein unter dem Sicherungselement liegender Teil des Riegels angeordnet sind, der beim Öffnen des Riegels am Sicherungselement vorbeigleitet und dieses nach einem Wiederverschließen des Riegels überdeckt. In dieser Ausführungsform gleitet das Sicherungselement beim Einschieben in die Halterung automatisch in den Spalt ein, wobei ein Teil des Sicherungselementes unter den Riegel gleitet, so daß der Riegel hier die Funktion eines Niederhalters übernimmt, während der andere Teil auf den Riegel aufgleitet. Beim Öffnen des Riegels wird dabei insbesondere bei einer das Sicherungselement beschädigenden Ausführung das Zerreißen der Sollbruchstelle erleichtert.

Bei einer anderen Ausführungsform kann der Niederhalter aber auch Teil der Halterung des Sicherungselementes sein.

Die nachfolgende Beschreibung einer bevorzugten Ausführungsform der Erfindung dient im Zusammenhang mit der Zeichnung der näheren Erläuterung. Es zeigen:
- Figur 1 :: eine perspektivische Draufsicht auf einen Sterilisierbehälter mit einem Riegelverschluß und einem kartenförmigen Sicherungselement;
- Figur 2 :: eine vergrößerte Teilansicht des parallel zur Behälterseitenwand geschnittenen Halters des Sicherungselementes;
- Figur 3 :: eine Schnittansicht längs Linie 3-3 mit einem Sicherungselement vor dem Öffnen des Riegels;
- Figur 4 :: eine Ansicht ähnlich Figur 3 mit dem Sicherungselement nach dem Öffnen und Wiederverschließen des Riegels;
- Figur 5 :: eine Ansicht ähnlich Figur 2 bei einem anderen bevorzugten Ausführungsbeispiel eines Riegelverschlusses und
- Figur 6 :: eine Ansicht ähnlich Figur 3 bei der Ausführungsform gemäß Figur 5.

Der in der Zeichnung dargestellte Sterilisierbehälter 1 umfaßt ein wannenförmiges Unterteil 2 sowie einen dichtend auf dieses Unterteil 2 aufsetzbaren Deckel 3. An einer vorderen Seitenwand und gegebenenfalls auch an einer in der Zeichnung nicht dargestellten gegenüberliegenden Seitenwand ist ein Verschluß 5 angeordnet, mit dem der Deckel 3 auf dem Unterteil 2 fixierbar ist. Dieser Verschluß umfaßt in dem dargestellten Ausführungsbeispiel einen um eine horizontale Achse verschwenkbar am Deckel 3 gelagerten Riegel 6, der in einer nach unten geklappten Schließstellung, in der er im wesentlichen parallel zur Seitenwand 4 angeordnet ist, ein Schließelement 7 an der Seitenwand 4 umgreift, während er dieses Schließelement 7 freigibt, wenn er um die horizontale Achse nach vorne ausgeschwenkt wird. Auf die Natur des Verschlusses kommt es für die vorliegende Erfindung nicht an, daher ist dieser Verschluß auch nicht näher erläutert. Selbstverständlich können hier die verschiedensten Schließmechanismen Verwendung finden, es ist auch möglich, daß der Riegel nicht am Deckel, sondern an der Seitenwand gelagert ist, wesentlich ist für die vorliegende Erfindung lediglich, daß der Verschluß einen zwischen einer Schließstellung und einer Offenstellung verschwenkbaren Riegel aufweist.

Neben dem Riegel 6 ist an der Seitenwand 4 eine Einschubhalterung 8 für ein kartenförmiges Sicherungselement 9 angeordnet, welches in dieser Einschubhalterung 8 parallel zur Seitenwand 4 verlaufend in einer Ebene angeordnet werden kann, die im wesentlichen coplanar zur Außenseite des in der Schließstellung stehenden Riegels 6 verläuft, gegebenenfalls könnte diese Ebene gegenüber der Außenseite des Riegels 6 auch geringfügig in Richtung auf den Behälter zurückgesetzt sein.

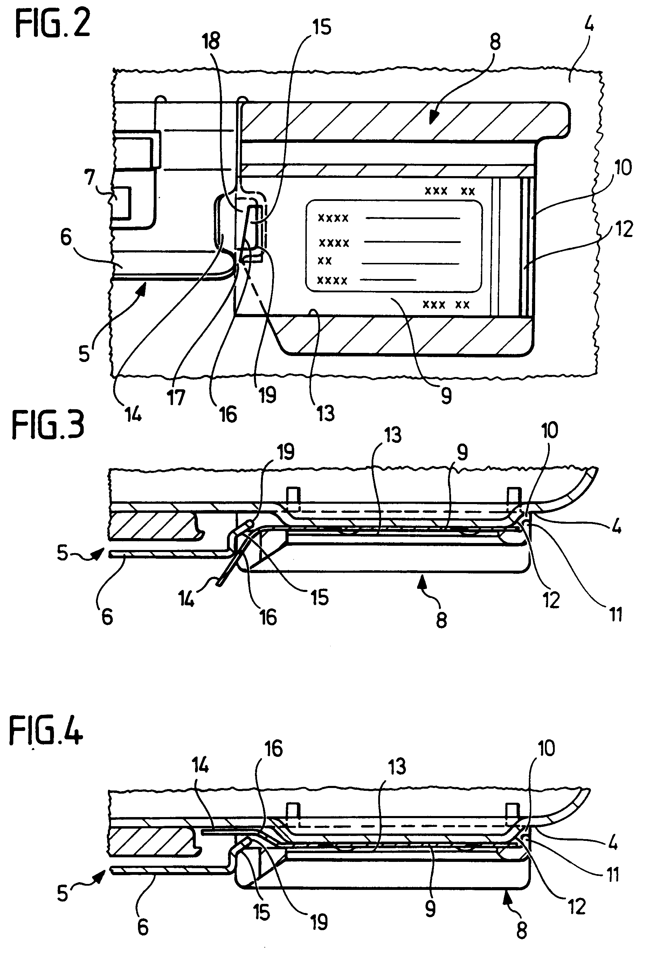

Die Einschubhalterung 8 weist auf der dem Riegel 6 abgewandten Seite einen Einschubschlitz 10 auf, der gegenüber der Ebene geringfügig in Richtung auf die Seitenwand 4 zurückgesetzt ist, in der das kartenförmige Sicherungselement 9 in der Einschubhalterung 8 gehalten wird (Figuren 3 und 4). Dies wird dadurch erreicht, daß die Einschubhalterung 8 an der dem Riegel 6 abgewandten Seite einen rechtwinklig umgebogenen und auf die Seitenwand 4 gerichteten Rand 11 aufweist. Unmittelbar neben dem Einschubschlitz 10 bildet die Seitenwand 4 eine Umlenkfläche 12 aus, die das kartenförmige Sicherungselement 9 nach dem Einschieben in den Einschubschlitz 10 in die Ebene umlenkt, in der das Sicherungselement 9 in der Einschubhalterung 8 gehalten wird.

Auf der dem Einschubschlitz 10 gegenüberliegenden und dem Riegel 6 damit unmittelbar benachbarten Seite ist in der Einschubhalterung 8 ebenfalls ein in der Ebene der Einschubhalterung 8 angeordneter Schlitz 13 angeordnet, durch den ein Teil 14 des kartenförmigen Sicherungselementes 9 aus der Einschubhalterung 8 hervorsteht und in die Bewegungsbahn des Riegels 6 eintaucht. Dieser vorstehende Teil 14 ist bei dem dargestellten Ausführungsbeispiel als seitlich von dem kartenförmigen Sicherungselement 9 abstehender Lappen ausgebildet, der bei geschlossenem Riegel 6 durch eine seitliche Umlenkfläche 15 am Riegel 6 vor den Riegel geschoben wird, wobei der Lappen in der aus Figur 3 ersichtlichen Weise schräg nach außen abgebogen wird.

Das kartenförmige Sicherungselement 9 weist unmittelbar neben dem als Lappen ausgebildeten Teil 14 eine Durchbrechung 16 auf, die zwischen dem Teil 14 und dem übrigen Bereich des Sicherungselementes 9 zwei Verbindungsstege ausbildet, nämlich einen schmalen Verbindungssteg 17 und einen breiten Verbindungssteg 18.

Die Umlenkfläche 15 am Riegel 6 ist ebenfalls lappenförmig ausgebildet und reicht mit ihrem äußeren Rand 19 bis in den Bereich der Durchbrechung 16 des in die Einschubhalterung 8 eingeschobenen Sicherungselementes 9 (Figur 2), so daß der Rand 19 beim Öffnen des Riegels in die Durchbrechung 16 eintritt.

Bei der Verwendung des Sterilisierbehälters wird zunächst der Behälter dadurch geschlossen, daß der Riegel 6 bei aufgelegtem Deckel 3 in die Schließstellung verschwenkt wird. Nach dem Verschließen des Behälters wird ein kartenförmiges Sicherungselement, auf dem Daten über den Inhalt des Behälters und die notwendigen Behandlungsschritte aufgetragen sein können, durch den seitlichen Einschubschlitz 10 in die Einschubhalterung 8 eingeschoben. Dabei gleitet der als Lappen ausgebildete Teil 14 an der Umlenkfläche 12 des Riegels entlang und legt sich in der aus Figur 3 ersichtlichen Weise vor den Riegel, wobei der Lappen schräg nach außen absteht. Er ist in dieser Stellung gut erkennbar und deutet an, daß der Riegel verschlossen geblieben ist.

Wird der Riegel geöffnet, greift der Rand 19 in die Durchbrechung 16 ein und reißt dadurch den schmalen Verbindungssteg 17 auf, während der breite Verbindungssteg 18 intakt bleibt. Bei einem weiteren Verschwenken des Riegels 6 in Offenstellung gleitet der nur noch über den breiten Verbindungssteg 18 mit dem Sicherungselement 9 verbundene Teil 14 an der Seitenkante des Riegels entlang und schwenkt hinter dem Riegel wieder in die Bewegungsbahn des Riegels ein, so daß beim erneuten Verschließen des Riegels dieser den Teil 14 mitnimmt und abdeckt, wie dies aus Figur 4 ersichtlich ist. Dadurch, daß der Lappen nicht mehr in der aus Figur 3 ersichtlichen Weise vor dem Riegel sichtbar ist, sondern in der aus Figur 4 ersichtlichen Weise vom Riegel abgedeckt wird, ist für den Benutzer ohne weiteres erkennbar, daß der Riegel unbeabsichtigt geöffnet worden ist.

Wird das Sicherungselement nach dem Öffnen des Riegels durch den Schlitz 13 aus der Einschubhalterung 8 herausgezogen, erkennt man durch den zerrissenen schmalen Verbindungssteg 17 sofort, daß im Verlauf der Behandlung eine unbeabsichtigte Öffnung des Riegels vorgenommen worden ist. Man erhält auf diese Weise eine vollständige Sicherheit über die Behandlungsgeschichte des Behälterinhaltes, insbesondere wenn gemäß einer bevorzugten Ausführungsform auf dem Sicherungselement 9 auch noch ein Sterilisationsindikatorfeld angeordnet wird, also ein Feld, das bei der Sterilisation einen Farbumschlag erfährt. Eine ordnungsgemäße Behandlung ist dann einmal am Behälter daran erkennbar, daß der Farbumschlag stattgefunden hat und daß der Lappen vor dem Riegel steht. An dem Sicherungselement 9 selbst erkennt man die ordnungsgemäße Behandlung durch den Farbumschlag und durch die Tatsache, daß sowohl der schmale Verbindungssteg 17 als auch der breite Verbindungssteg 18 intakt sind.

Es ist auch nicht möglich, den Riegel zu öffnen, ohne das Sicherungselement zu beschädigen, da das Sicherungselement nur bei geöffnetem Riegel aus der Einschubhalterung entfernt werden kann, und zwar nur durch den Schlitz 13, da ein Ausschieben in Gegenrichtung wegen der seitlichen Versetzung des Einschubschlitzes 10 nicht möglich ist. Damit ist insgesamt eine größtmögliche Sicherheit gewährleistet.

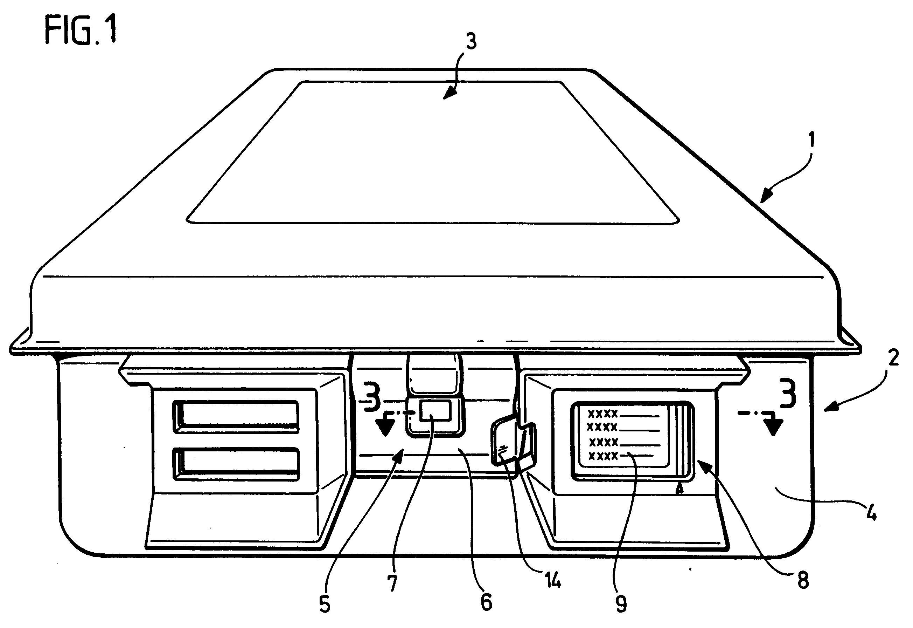

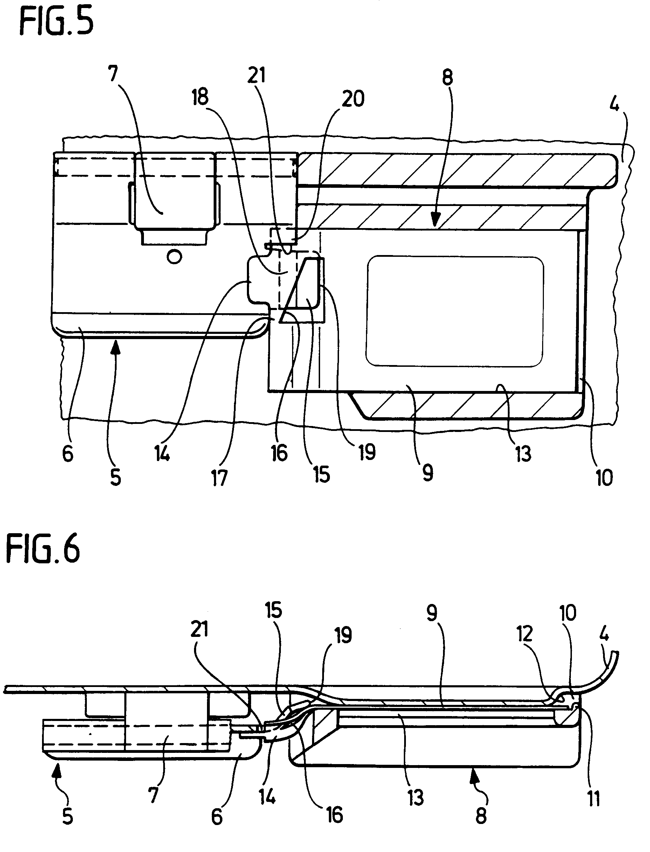

Bei dem in den Figuren 5 und 6 dargestellten Ausführungsbeispiel sind gegenüber dem Ausführungsbeispiel der Figuren 1 bis 4 nur geringfügige Änderungen vorgenommen, einander entsprechende Teile tragen daher dieselben Bezugszeichen.

Bei dem Ausführungsbeispiel der Figuren 5 und 6 schließt sich an die das Sicherungselement 9 untergreifende Umlenkfläche 15 eine als Niederhalter 20 wirkende Fläche an, die von der Umlenkfläche 15 durch einen vom Außenrand des Riegels 6 nach innen parallel zur Einschubrichtung des Sicherungselementes 9 in der Halterung 8 verlaufenden Spalt 21 getrennt ist. Beim Einschieben des Sicherungselementes 9 in die Einschubhalterung 8 lenkt die Umlenkfläche 15 das Sicherungselement 9 im Bereich der Umlenkfläche 15 vor den Riegel, im Bereich des Niederhalters 20 jedoch hinter den Riegel, wobei das Sicherungselement 9 dabei in den Spalt 21 eintritt. Dabei ist die Anordnung so gewählt, daß das Sicherungselement 9 nach dem Einschieben im Bereich des Spaltes 21 nach außen vorgespannt wird.

Solange das Sicherungselement in der in Figur 5 gezeigten Weise unbeschädigt ist, führt diese Vorspannung allenfalls zu einer geringfügigen Verformung des Sicherungselementes, auch der mit dem übrigen Sicherungselement über die Stege 17 und 18 verbundene Teil des Sicherungselementes liegt dadurch im wesentlichen parallel zum Riegel vor diesem, wie dies in Figur 5 gezeigt ist.

Wenn jedoch der schmale Steg 17 des Sicherungselementes 9 aus irgendeinem Grunde beschädigt ist, führt die Vorspannung im Bereich des Spaltes 21 dazu, daß der jetzt nur noch über den breiten Steg 18 mit dem Sicherungselement 9 verbundene Teil nach außen absteht, d.h. es ist sofort erkennbar, daß das Sicherungselement beschädigt ist. Dadurch ergibt sich auch eine Sicherung gegen die Verwendung eines beschädigten Sicherungselementes, d. h. man erhält eine zusätzliche Überprüfung darauf, ob die in die Halterung eingesetzten Sicherungselemente einwandfrei sind.

## Patentansprüche

1. Sterilisierbehälter für chirurgische Instrumente oder dergleichen mit einem Unterteil, einem abgedichtet auf dieses aufsetzbaren Deckel, einem den Deckel am Unterteil festlegenden Riegel, der zwischen einer Schließstellung und einer Offenstellung verschwenkbar ist, und mit einem kartenähnlichen Sicherungselement, das am Behälter festlegbar ist und nahe der Schließstellung des Riegels in die Bewegungsbahn des Riegels eintaucht,
dadurch gekennzeichnet, daß der in die Bewegungsbahn des Riegels (6) eintauchende Teil (14) des Sicherungselementes (9) aus der Ebene des Sicherungselementes (9) elastisch abbiegbar ist und nur so tief in die Bewegungsbahn des Riegels (6) eintaucht, daß er beim Verschwenken des Riegels (6) in die Offenstellung hinter den Riegel (6) zurückgleitet und beim Schließen des Riegels (6) von diesem mitgenommen wird und in der Schließstellung des Riegels (6) von diesem abgedeckt wird.

2. Sterilisierbehälter nach Anspruch 1, dadurch gekennzeichnet, daß der in die Bewegungsbahn des Riegels (6) eintauchende Teil (14) ein seitlich von dem kartenähnlichen Sicherungselement (9) abstehender Lappen ist.

3. Sterilisierbehälter nach einem der Ansprüche 1 oder 2, dadurch gekennzeichnet, daß neben dem Riegel (6) eine Halterung (8) für das kartenähnliche Sicherungselement (9) angeordnet ist, in welche das Sicherungselement (9) nach dem Schließen des Riegels (6) derart einlegbar oder einschiebbar ist, daß der in die Bewegungsbahn des Riegels (6) eintauchende Teil (14) unmittelbar vor dem Riegel (6) positioniert ist.

4. Sterilisierbehälter nach Anspruch 3, dadurch gekennzeichnet, daß der in die Bewegungsbahn des Riegels (6) eintauchende Teil (14) nach dem Einlegen oder Einschieben in die Halterung (8) durch die Anlage am Riegel (6) nach vorne aus der Ebene des kartenförmigen Sicherungselementes (9) abgebogen ist.

5. Sterilisierbehälter nach einem der Ansprüche 3 oder 4, dadurch gekennzeichnet, daß der Riegel (6) eine Umlenkfläche (12) aufweist, an der der in die Bewegungsbahn des Riegels (6) eintauchende Teil (14) des kartenförmigen Sicherungselementes (9) beim Einlegen oder Einschieben in die Halterung (8) vor den Riegel (6) gelenkt wird.

6. Sterilisierbehälter nach einem der voranstehenden Ansprüche, dadurch gekennzeichnet, daß die Halterung (8) auf ihrer dem Riegel (6) abgewandten Seite einen Einführschlitz (10) für das Sicherungselement (9) aufweist.

7. Sterilisierbehälter nach Anspruch 6, dadurch gekennzeichnet, daß der Einführschlitz (10) gegenüber der Ebene, in welcher das Sicherungselement (9) im Halter (8) positionierbar ist, seitlich versetzt ist.

8. Sterilisierbehälter nach einem der voranstehenden Ansprüche, dadurch gekennzeichnet, daß der in die Bewegungsbahn des Riegels (6) eintauchende Teil (14) des kartenförmigen Sicherungselementes (9) mittels einer Sollbruchstelle (Steg 17) und mittels einer festen flexiblen Verbindung (Steg 18) mit dem übrigen Sicherungselement (9) verbunden ist und daß die Sollbruchstelle so ausgebildet und positioniert ist, daß sie bricht, wenn der Riegel (6) den vor ihm liegenden Teil (14) des Sicherungselementes (9) beim Verschwenken in Offenstellung mitnimmt, während die festere Verbindung erhalten bleibt.

9. Sterilisierbehälter nach Anspruch 8, dadurch gekennzeichnet, daß der in die Bewegungsbahn des Riegels (6) eintauchende Teil (14) über zwei verschieden breite Verbindungsstege (17, 18) mit dem übrigen Sicherungselement (9) verbunden ist.

10. Sterilisierbehälter nach Anspruch 8, dadurch gekennzeichnet, daß im Sicherungselement (9) neben dem in die Bewegungsbahn des Riegels (6) eintauchenden Teil (14) eine Durchbrechung (16) angeordnet ist, die zwischen dem in die Bewegungsbahn eintauchenden Teil (14) und dem übrigen Sicherungselement (9) einen breiten und einen schmalen Verbindungssteg (18 beziehungsweise 17) ausbildet.

11. Sterilisierbehälter nach einem der Ansprüche 9 oder 10, dadurch gekennzeichnet, daß der Riegel (6) einen seitliche Fortsatz (Rand 19) trägt, der beim Verschwenken des Riegels (6) in Offenstellung zwischen die Verbindungsstege (17, 18) eingreift und beim weiteren Verschwenken dadurch den schmaleren Verbindungssteg (17) aufreißt.

12. Sterilisierbehälter nach einem der Ansprüche 1 bis 7, dadurch gekennzeichnet, daß der Riegel (6) ein Markierglied trägt, das beim Verschwenken des Riegels aus der Schließstellung in die Offenstellung das Sicherungselement durchdringt und dadurch beschädigt.

13. Sterilisierbehälter nach einem der Ansprüche 3 bis 12, dadurch gekennzeichnet, daß neben dem vor dem Riegel (6) positionierten Teil des Sicherungselementes (9) ein Niederhalter (20) angeordnet ist, der sich vor dem Sicherungselement (9) befindet und dieses in Richtung auf den Sterilisierbehälter (1) festlegt.

14. Sterilisierbehälter nach Anspruch 13, dadurch gekennzeichnet, daß der Niederhalter (20) das Sicherungselement (9) in einer Position festlegt, in der das Sicherungselement (9) zwischen dem vom Niederhalter (20) überdeckten Bereich und dem vor dem Riegel (6) liegenden Bereich vom Sterilisierbehälter (1) nach außen weisend vorgespannt wird.

15. Sterilisierbehälter nach Anspruch 13 oder 14, dadurch gekennzeichnet, daß der Niederhalter (20) Teil des Riegels (6) ist.

16. Sterilisierbehälter nach Anspruch 15, dadurch gekennzeichnet, daß im Riegel (6) ein parallel zur Einschubrichtung des Sicherungselementes (9) verlaufender Spalt (21) angeordnet ist, an dessen einer Seite der das Sicherungselement (9) überdeckende Niederhalter (20) und an dessen anderer Seite ein unter dem Sicherungselement (9) liegender Teil des Riegels angeordnet sind, der beim Öffnen des Riegels (6) am Sicherungselement (9) vorbeigleitet und dieses nach einem Wiederverschließen des Riegels (6) überdeckt.

17. Sterilisierbehälter nach einem der Ansprüche 13 oder 14, dadurch gekennzeichnet, daß der Niederhalter Teil der Halterung (8) des Sicherungselementes (9) ist.

## Claims

1. A sterilisation container for surgical instruments or the like, having a base, a lid which can be placed onto this base so as to be sealed, a locking element fixing the lid on the base, which locking element can be swung between a closed-position and an open-position, and having a card-like security element, which can be fixed on the container and which projects into the path of motion of the locking element close to its closed-position,
characterised in that the part (14) of the security element (9) which projects into the path of motion of the locking element (6) is resiliently bendable out of the plane of the security element (9) and projects only so far into the path of motion of the locking element (6) that, when the locking element (6) is swung into the open-position, the part slides back behind the locking element (6) and when the locking element (6) is closed, the part is carried by this and covered by the locking element (6) in the closed-position thereof.

2. A sterilisation container according to claim 1, characterised in that part (14) which projects into the path of motion of the locking element (6) is a tab which projects out laterally from card-like security element (9).

3. A sterilisation container according to any one of claims 1 or 2, characterised in that a holder (8) for the card-like security element (9) is arranged next to the locking element (6), in which holder the security element (9) can be inserted or slid-in, after the locking element (6) is closed, in such a way that the part (14) which projects into the path of motion of the locking element (6) is positioned directly in front of the locking element (6).

4. A sterilisation container according to claim 3, characterised in that the part (14) which projects into the path of motion of the locking element (6), is bent forwards out of the plane of the card-like security element (9) by leaning against the locking element (6), after it has been inserted or slid into the holder (8).

5. A sterilisation container according to any one of claims 3 or 4, characterised in that the locking element (6) has a guide face (12), on which the part (14) of the card-like security element which projects into the path of motion of the locking element (6), on insertion or sliding into the holder (8), is guided in front of the locking element (6).

6. A sterilisation container according to any one of the preceding claims, characterised in that the holder (8), on its side remote from the locking element (6), has an insertion slot (10) for the security element (9).

7. A sterilisation container according to claim 6, characterised in that the insertion slot (10) is laterally offset against the plane in which the security element (9) can be positioned in the holder (8).

8. A sterilisation container according to any one of the preceding claims, characterised in that the part (14) of the card-shaped security element (9) which projects into the path of motion of the locking element (6) is connected with the rest of the security element (9) by means of a break-off point (ridge 17) and by means of a secure, flexible connection (ridge 18), and in that the break-off point is formed and positioned in such a way that it breaks when the locking element (6), when swung into an open position, takes with it the part (14) of the security element (9) located in front of it, while the more secure connection remains intact.

9. A sterilisation container according to claim 8, characterised in that the part (14) which projects into the path of motion of the locking element (6) is connected to the rest of the security element (9) by two connecting ridges (17, 18) of different widths.

10. A sterilisation container according to claim 8, characterised in that an opening (16) is arranged in the security element (9) next to the part (14) which projects into the path of motion of the locking element (6), which opening (16) forms a wide and a narrow connecting ridge (18 or 17) between the part (14) which projects into the path of motion and the rest of the security element (9).

11. A sterilisation container according to any one of claims 9 or 10, characterised in that the locking element (6) has a lateral prolongation (edge 19), which when the locking element (6) is swung into an open position, engages between the connecting ridges (17, 18), and on further swinging, tears open the narrower connecting ridge (17).

12. A sterilisation container according to any one of claims 1 to 7, characterised in that the locking element (6) has a marking member which, when the locking element is swung out of the closed-position into the open-position, pierces through and thus damages the security element.

13. A sterilisation container according to any one of claims 3 to 12, characterised in that a retainer (20) is arranged next to the part of the security element (9) positioned in front of the locking element (6), which retainer is situated in front of the security element (9) and fixes this in the direction of the sterilisation container (1).

14. A sterilisation container according to claim 13, characterised in that the retainer (20) fixes the security element (9) in a position in which the security element (9) is urged to point outwards between the region overlapped by the retainer (20) and the region of the sterilisation container (1) situated in front of the locking element (6).

15. A sterilisation container according to claim 13 or 14, characterised in that the retainer (20) is part of the locking element (6).

16. A sterilisation container according to claim 15, characterised in that in the locking element (6), is arranged a gap (21), which extends parallel to the direction of insertion of the security element (9), there being arranged on the one side of which gap, the retainer (20) overlapping the security element (9), and on the other side of which gap, a part of the locking element situated under the security element (9), which part slides past the security element (9) when the locking element (6) is opened, and overlaps the security element when the locking element (6) is closed again.

17. A sterilisation container according to any one of claims 13 or 14, characterised in that the retainer is part of the holder (8) of the security element (9).

## Revendications

1. Récipient de stérilisation pour des instruments chirurgicaux ou similaires, comportant une partie inférieure, un couvercle susceptible d'être posé avec étanchéité sur celle-ci, un verrou qui fixe le couvercle sur la partie inférieure et qui est capable de basculer entre une position de fermeture et une position d'ouverture, et comportant un élément de sécurité en forme de carte qui est susceptible d'être fixé sur le récipient et qui plonge dans le trajet de déplacement du verrou à proximité de la position de fermeture du verrou, caractérisé en ce que la partie de l'élément de sécurité qui plonge dans le trajet de déplacement du verrou (6) est flexible élastiquement hors du plan de l'élément de sécurité (9) et en ce qu'elle plonge uniquement aussi loin dans le trajet de déplacement du verrou (6), qu'elle coulisse en retour derrière le verrou (6) lors du basculement du verrou (6) dans la position d'ouverture, et qu'elle est entraînée par le verrou (6) lors de la fermeture de celui-ci et est recouverte par le verrou (6) dans la position de fermeture de celui-ci.

2. Récipient de stérilisation selon la revendication 1, caractérisé en ce que la partie (14) plongeant dans le trajet de déplacement du verrou (6) est une patte latéralement en dépassement depuis l'élément de sécurité (9) en forme de carte.

3. Récipient de stérilisation selon l'une ou l'autre des revendications 1 et 2, caractérisé en ce qu'il est prévu à côté du verrou (6) une monture (8) pour l'élément de sécurité (9) en forme de carte, dans laquelle l'élément de sécurité (9) peut être mis en place ou enfilé après la fermeture du verrou (6), de telle sorte que la partie (14) plongeant dans le trajet de déplacement du verrou (6) est positionnée directement devant le verrou (6).

4. Récipient de stérilisation selon la revendication 3, caractérisé en ce que la partie (14) plongeant dans le trajet de déplacement du verrou (6) est fléchie, après sa mise en place ou son enfilement dans la monture (8), par appui contre le verrou (6) vers l'avant hors du plan de l'élément de sécurité (9) en forme de carte.

5. Récipient de stérilisation selon l'une ou l'autre des revendications 3 et 4, caractérisé en ce que le verrou (6) comporte une surface de renvoi (12) sur laquelle la partie (14) de l'élément de sécurité (9) en forme de carte qui plonge dans le trajet de déplacement du verrou (6) est déviée en avant du verrou (6) lors de la mise en place ou de l'enfilement dans la monture (8).

6. Récipient de stérilisation selon l'une quelconque des revendications précédentes, caractérisé en ce que la monture (8) comporte sur son côté détourné du verrou (6) une fente d'introduction (10) pour l'élément de sécurité (9).

7. Récipient de stérilisation selon la revendication 6, caractérisé en ce que la fente d'introduction (10) est latéralement décalée par rapport au plan dans lequel l'élément de sécurité (9) peut être positionné dans la monture (8).

8. Récipient de stérilisation selon l'une quelconque des revendications précédentes, caractérisé en ce que la partie (14) de l'élément de sécurité (9) en forme de carte qui plonge dans le trajet de déplacement du verrou (6), est reliée au reste de l'élément de sécurité (9) au moyen d'un emplacement destiné à la rupture (barrette 17) et au moyen d'une liaison ferme et flexible (barrette 18), et en ce que l'emplacement destiné à la rupture est réalisé et positionné de telle sorte qu'il se rompt lorsque le verrou (6) entraîne la partie (14) de l'élément de sécurité (9) située devant celui-ci pendant le basculement dans la position d'ouverture, tandis que la liaison ferme reste maintenue.

9. Récipient de stérilisation selon la revendication 8, caractérisé en ce que la partie (14) plongeant dans le trajet de déplacement du verrou (6) est reliée au reste de l'élément de sécurité (9) par l'intermédiaire de deux barrettes de liaison (17, 18) de différentes largeurs.

10. Récipient selon la revendication 8, caractérisé en ce qu'il est prévu dans l'élément de sécurité (9), à côté de la partie (14) plongeant dans le trajet de déplacement du verrou (6), une traversée (16) qui forme entre la partie (14) plongeant dans le trajet de déplacement et le reste de l'élément de sécurité (9) une barrette de liaison large (18) et une barrette de liaison étroite (17).

11. Récipient de stérilisation selon l'une ou l'autre des revendications 9 et 10, caractérisé en ce que le verrou (6) porte un prolongement latéral (bordure 19) qui s'engage entre les barrettes de liaison (17, 18) lors du basculement du verrou (6) dans la position d'ouverture, et qui déchire ainsi la barrette de liaison plus étroite (17) lors de la poursuite du basculement.

12. Récipient de stérilisation selon l'une quelconques des revendications 1 à 7, caractérisé en ce que le verrou (6) porte un organe de marquage qui traverse l'élément de sécurité (9) et qui l'endommage ainsi lors du basculement du verrou (6) hors de la position de fermeture jusque dans la position d'ouverture.

13. Récipient de stérilisation selon l'une quelconque des revendications 3 à 12, caractérisé en ce qu'il est prévu, à côté de la partie de l'élément de sécurité (9), positionnée devant le verrou (6), un élément de maintien en position basse (20) qui se trouve en avant de l'élément de sécurité (9) et qui fixe celui-ci en direction du récipient de stérilisation (1).

14. Récipient selon la revendication 13, caractérisé en ce que l'élément de maintien en position basse (20) fixe l'élément de sécurité (9) dans une position dans laquelle l'élément de sécurité (9) est mis sous précontrainte en étant dirigé vers l'extérieur entre la région recouverte par l'élément de maintien en position basse (20) et la région située devant le verrou (6) du récipient de stérilisation (1).

15. Récipient de stérilisation selon l'une ou l'autre des revendications 13 et 14, caractérisé en ce que l'élément de maintien en position basse (20) fait partie du verrou (6).

16. Récipient de stérilisation selon la revendication 15, caractérisé en ce qu'il est prévu dans le verrou (6) une fente (21) qui s'étend parallèlement à la direction d'enfilement de l'élément de sécurité (9) et sur un côté de cette fente est agencé l'élément de maintien en position basse (20) recouvrant l'élément de sécurité (9) et sur l'autre côté de celle-ci est agencée une partie du verrou située au-dessous de l'élément de sécurité (9), qui passe en coulissant le long de l'élément de sécurité (9) lors de l'ouverture du verrou (6) et recouvre celui-ci après une nouvelle fermeture du verrou (6).

17. Récipient de stérilisation selon l'une ou l'autre des revendications 13 ou 14, caractérisé en ce que l'élément de maintien en position basse fait partie de la monture (8) de l'élément de sécurité (9).
